(19) Europäisches Patentamt
European Patent Office
Office européen des brevets

(11) Publication number : **0 350 422 B1**

# (12) EUROPEAN PATENT SPECIFICATION

(45) Date of publication of patent specification : **16.09.92 Bulletin 92/38**

(51) Int. Cl.$^5$ : **C07D 233/64,** A61K 31/415

(21) Application number : **89500060.2**

(22) Date of filing : **23.05.89**

## (54) Propargylguanidine derivatives and process for the preparation thereof.

(30) Priority : **06.07.88 ES 8802117**

(43) Date of publication of application : **10.01.90 Bulletin 90/02**

(45) Publication of the grant of the patent : **16.09.92 Bulletin 92/38**

(84) Designated Contracting States : **AT BE CH DE FR GB IT LI NL SE**

(56) References cited :
**FR-A- 2 401 143**
**US-A- 4 381 395**

(73) Proprietor : **LABORATORIOS VINAS S.A.**
**Provenza 386**
**E-08025 Barcelona (ES)**

(72) Inventor : **Buxade Vinas, Antonio**
**Urgell 230**
**E-08036 Barcelona (ES)**

(74) Representative : **Curell Sunol, Jorge et al**
**c/o Dr. Ing. M. Curell Sunol I.I. S.L. Passeig de Gràcia 65 bis**
**E-08008 Barcelona (ES)**

EP 0 350 422 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to propargylguanidine derivatives of the general formula I:

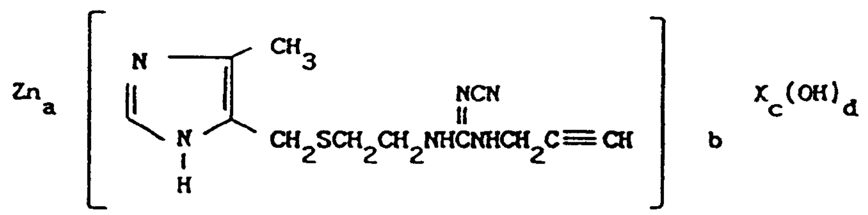

(I)

where:

X is an anion of pharmaceutically acceptable acids;
a is an integer from 1 to 5;
b is an integer from 1 to 7;
c is O or an integer from 1 to 4; and
d is 2a - c for monovalent acid anions,
is a - c for divalent acid anions, and
is O or 2 when c = O.

The invention also relates to a process for the preparation of the derivatives of formula I, which have antiulcer properties.

Chronic gastric and duodenal ulcers are frequent disorders for which a wide range of treatments exists, including dietary measures, treatment with drugs and surgery. Among these, special attention has been paid in recent years to treatment with secretion inhibitors, one of the secretion inhibitors currently under development being N-cyano-N′-2-((4-methyl-5-imidazolyl)methylthio)ethyl-N″-propargylguanidine of formula II (hereinafter abbreviatedly called "Et").

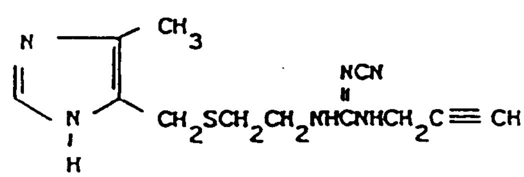

(II)

is disclosed in FR-A- 2 4O1 143.

The propargylguanidine derivatives of the present invention provide advantages on improving the antiulcer properties of the compound Et.

In the propargylguanidine derivatives of formula I, X may be an anion of pharmaceutically acceptable inorganic acids such as hydrochloric acid, sulphuric acid, phosphoric acid, hydrobromic acid, hydroiodic acid and others; or an anion of non-toxic organic acids, such mono- and dicarboxylic aliphatic acids, alkanoic acids with phenyl substituents, hydroxyalkanoic acids, alkanodioic acids, aromatic acids, aromatic and aliphatic sulphonic acids and others.

Therefore, X may be chloride, bromide, iodide, fluoride, sulphate, phosphate, chlorate, nitrate, sulphamate, maleate, fumarate, succinate, oxalate, acetate, acexamate, tartrate, citrate, camphorsulphonate, mandelate, butyno-1,4-dioate, benzoate, chlorobenzoate, methylbenzoate, hydroxybenzoate, methoxybenzoate, benzene sulphonate, toluene sulphonate, phenyl acetate, salicylate, betahydroxybutyrate, glycolate or methane sulphonate.

The process of the invention is characterised in that the compound of formula II is reacted with an X anion containing zinc compound, or with an organozinc.

Said zinc compound is preferably an organic or inorganic zinc salt and the reaction is conducted in a polar organic solvent such as dimethylformamide, dimethyl sulphoxide, 1,4-dioxane, acetone, low molecular weight alcohols, etc. or in an organic aqueous mixture; preferably an alcohol will be used. The reaction temperature may reach up to the boiling point of the solvent, preferably from room temperature to 6O°C. According to the type of compound it is wanted to isolate, an alkali or alkali earth hydroxide may be added at the end to alkaline pH, preferably to pH 8. Water may also be added when the reaction has been conducted in an organic solvent

alone.

The compounds are obtained in solid form or in the form of a thick oil which, after removal of the solvent at reduced pressure, becomes a solid.

The compounds of formula I have shown a potent antiulcer activity. Thus, inhibition of histamine-induced gastric secretion in rats "in vivo" after intraperitoneal administration of the present Zn-propargylguanidine compounds at a dose of 1 to 1O mg/kg, reaches pH recovery levels of 95%. The antiulcer activity in ethanol-induced ulcers in rats has proved to be satisfactory at dose levels between 1OO and 25O mg/kg body weight p.o., and in certain cases total ulcer inhibition has been observed.

To facilitate the explanation, the invention is illustrated by the following example.

## EXAMPLE

### Preparation of the $Zn(Et)Cl_2$

A solution of 3.5 g of Et was added slowly over a solution of 1.72 g of zinc chloride in 3O ml of ethanol. During the addition, an oil was formed, which was separated by decantation of the solvent. 25 ml of ethanol were added and after stirring for 15 minutes, the solvent was removed again by decantation. After drying at reduced pressure at a temperature of 7O°C, 4.4 g of a powdery product were obtained.

Elementary analysis:

Calculated for $C_{12}H_{16}Cl_2N_6SZn$:

C: 34.93; H: 3.91; Cl: 17.18; N: 2O.37; S: 7.77 and Zn: 15.85

Found:

C: 34.57; H: 4.25; Cl: 16.75; N: 2O.O5; S: 7.55 and Zn: 15.4O

IR(KBr) : 3411, 22O8, 1616, 15O7, 1458, 1437, 1228, 626 $cm^{-1}$.

$^1H$ NMR ($CD_3SOCD_3$, TMS as internal standard). δ: 2.17 (singlet, 3H); 2.4-2.7 (complex absorption, 2H); 3.13 (triplet, 1H); 3.1-3.5 (complex absorption, 2H); 3.77 (singlet, 2H); 3.8-4.O (complex absorption, 2H); 7.13 (triplet, 1H), 7.42 (triplet, 1H) and 7.82 (singlet, 1H).

There are described below experiments which show the therapeutic activity of $Zn(Et)Cl_2$, referred to hereinafter as LV-198.

### Antiulcer activity of compound LV-198

The antiulcer activity of LV-198 was studied on different experimental models, some of which are described below:

A) Model of histamine-induced gastric acid secretion in rat "in vivo".

This activity was measured in Wistar rats having a body weight of 24O ± 2O g. An endovenous perfusion of a histamine solution stimulated gastric secretion, producing a reduction in the intragastric pH. Thereafter LV-198 was administered intraperitoneally and the increase of the gastric pH was evaluated. Thus, the pH recovery was 8O% at a dose of 5 mg/kg.

B) Necrotising agent model:

Using the necrotising agent model, ethanol in this case, described by Robert el al. (Gastroenterology, 77: 433, 1979), the activity of LV-198 was compared with that of Et at equimolar dose levels and a control group which was administered vehicle.

The table below gives the lesion indices (in ulcerated area in mm) of the three groups studied, as well as the percentage inhibition of the ulcers of the treated groups over the control group (mean values ± standard error of the mean).

| | mm lesions | % inhibition |
|---|---|---|
| Control | 36.5 $\pm$ 10.0 | --- |
| Et | 31.8 $\pm$ 17.8 | 12.9 |
| LV-198 (200 mg/kg) | 2.2 $\pm$ 1.9[a,b] | 93.4 |

t test: a $p < 0.01$ vs control: b $p < 0.05$ versus Et

As may be seen, Et does not inhibit ulcers induced by absolute ethanol, while practically total inhibition is obtained with LV-198.

## Claims

1.- Propargylguanidine derivatives of the general formula I:

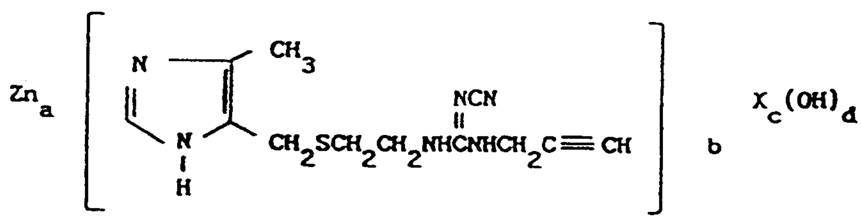

(I)

where:

X is an anion of pharmaceutically acceptable acids;
a is an integer from 1 to 5;
b is an integer from 1 to 7;
c is O or an integer from 1 to 4; and
d is 2a - c for monovalent acid anions,
is a - c for divalent acid anions, and
is O or 2 when c = O.

2.- The derivatives of claim 1, characterised in that X is selected from the group formed by chloride, bromide, iodide, fluoride, sulphate, phosphate, chlorate, nitrate, sulphamate, maleate, fumarate, succinate, oxalate, acetate, acexamate, tartrate, citrate, camphorsulphonate, mandelate, butyno-1,4-dioate, benzoate, chlorobenzoate, methylbenzoate, hydroxybenzoate, methoxybenzoate, benzene sulphonate, toluene sulphonate, phenyl acetate, salicylate, betahydroxybutyrate, glycolate and methane sulphonate.

3.- The derivatives of claim 1, characterised in that X is the chloride anion, a = 1, b = 1, c = 2 and d = O.

4.- A process for the preparation of propargylguanidine derivatives of the general formula I:

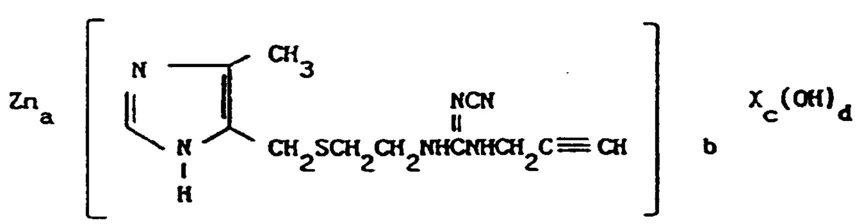

(I)

where:

X is an anion of pharmaceutically acceptable acids;
a is an integer from 1 to 5;

b is an integer from 1 to 7;
c is O or an integer from 1 to 4; and
d is 2a - c for monovalent acid anions,
is a - c for divalent acid anions, and
is O or 2 when c = O,

characterised in that N-cyano-N'-(2-((4-methyl-5-imidazolyl)methylthio)ethyl)-N"-propargylguanidine of formula II

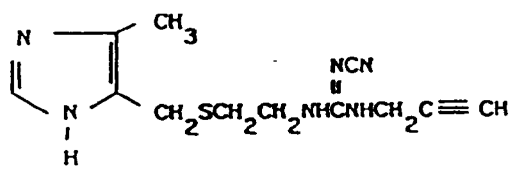

(II)

is reacted with an X anion containing zinc compound or an organozinc.

5.- The process of claim 4, characterised in that said compound is an X anion containing zinc salt.

6.- The process of claim 4, characterised in that X is selected from the group formed by chloride, bromide, iodide, fluoride, sulphate, phosphate, chlorate, nitrate, sulphamate, maleate, fumarate, succinate, oxalate, acetate, acexamate, tartrate, citrate, camphorsulphonate, mandelate, butyno-1,4-dioate, benzoate, chlorobenzoate, methylbenzoate, hydroxybenzoate, methoxybenzoate, benzene sulphonate, toluene sulphonate, phenyl acetate, salicylate, betahydroxybutyrate, glycolate and methane sulphonate.

7.- The process of claim 5, characterised in that said salt is zinc chloride.

8.The process of any one of claims 1-7, characterised in that the reaction is conducted in a polar solvent.

9.- The process of claim 8, characterised in that said solvent is a low molecular weight alcohol.

1O.- The process of claim 8, characterised in that said solvent is selected from the group formed by dimethylformamide, dimethylsulphoxide, 1,4- dioxane, acetone, or aqueous mixtures thereof.

## Patentansprüche

1. Propargylguanidin-Derivate mit der allgemeinen Formel I:

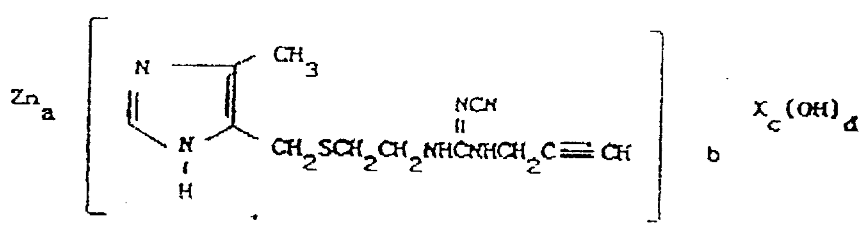

(I)

worin:

X ein Anion einer pharmazeutisch annehmbaren Säure ist;
a eine ganze Zahl von 1 bis 5 ist;
b eine ganze Zahl von 1 bis 7 ist;
c gleich 0 oder eine ganze Zahl von 1 bis 4 ist; und
d bei monovalenten Säureanionen gleich 2a - c ist,
bei divalenten Säureanionen gleich a - c ist, und
gleich 0 oder 2 ist, wenn c = 0.

2. Derivate nach Anspruch 1, **dadurch gekennzeichnet**, daß X aus der durch Chlorid, Bromid, Jodid, Fluorid, Sulfat, Phosphat, Chlorat, Nitrat, Sulfamat, Maleat, Fumarat, Succinat, Oxalat, Acetat, Acexamat, Tartrat, Zitrat, Camphersulfonat, Mandelat, Butyro-1,4-dioat, Benzoat, Chlorobenzoat, Methylbenzoat, Hydroxybenzoat, Methoxybenzoat, Benzolsulfonat, Toluolsulfonat, Phenylacetat, Salicylat, beta-Hydroxybutyrat, Glycolat und Methansulfonat gebildenten Gruppe ausgewählt ist.

3. Derivate nach Anspruch 1, **dadurch gekennzeichnet,** daß X das Chloridanion ist, und a = 1, b = 1, c =

2 und d = 0.

4. Verfahren zur Herstellung von Propargylguanidin-Derivaten der allgemeinen Formel I:

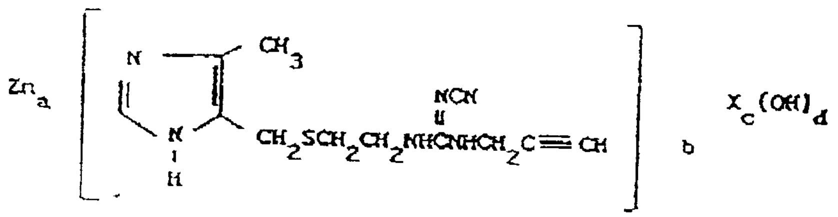

(I)

worin:

X ein Anion von pharmazeutisch annehmbaren Säuren ist;
a eine ganze Zahl von 1 bis 5 ist;
b eine ganze Zahl von 1 bis 7 ist;
c gleich 0 oder eine ganze Zahl von 1 bis 4 ist; und
d bei monovalenten Säureanionen gleich 2a - c ist,
bei divalenten Säureanionen gleich a - c ist, und
gleich 0 oder 2 ist, wenn c = 0,

**dadurch gekennzeichnet,** daß N-Cyano-N′-(2-((4-methyl-5-imidazolyl)methylthio)ethyl)-N″-propargylguanidin der Formel II

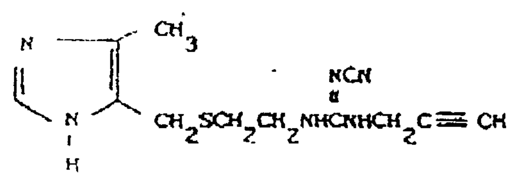

(II)

mit einer ein X-Anion enthaltenden Zinkverbindung oder einem Organozink umgesetzt wird.

5. Verfahren nach Anspruch 4, **dadurch gekennzeichnet,** daß als Verbindung ein X-Anion enthaltendes Zinksalz verwendet wird.

6. Verfahren nach Anspruch 4, **dadurch gekennzeichnet,** daß X aus einer aus Chlorid, Bromid, Jodid, Fluorid, Sulfat, Phosphat, Chlorat, Nitrat, Sulfamat, Maleat, Fumarat, Succinat, Oxalat, Acetat, Acexamat, Tartrat, Zitrat, Camphersulfonat, Mandelat, Butylo-1,4-dioat, Benzoat, Chlorobenzoat, Methylbenzoat, Hydroxybenzoat, Methoxybenzoat, Benzolsulfonat, Toluolsulfonat, Phenylacetat, Salicylat, beta-Hydroxybutyrat, Glycolat und Methansulfonat gebildeten Gruppe ausgewählt wird.

7. Verfahren nach Anspruch 5, **dadurch gekennzeichnet,** daß als Salz Zinkchlorid verwendet wird.

8. Verfahren nach einem der Ansprüche 1 bis 7, **dadurch gekennzeichnet,** daß die Reaktion in einem polaren Lösungsmittel durchgeführt wird.

9. Verfahren nach Anspruch 8, **dadurch gekennzeichnet,** daß als Lösungsmittel ein niedermolekularer Alkohol verwendet wird.

10. Verfahren nach Anspruch 9, **dadurch gekennzeichnet,** daß das Lösungsmittel aus einer aus Dimethylformamid, Dimethylsufoxid, 1,4-Dioxan, Aceton oder Mischungen davon gebildeten Gruppe ausgewählt wird.

## Revendications

1. Dérivés de propargylguanidine de formule générale I:

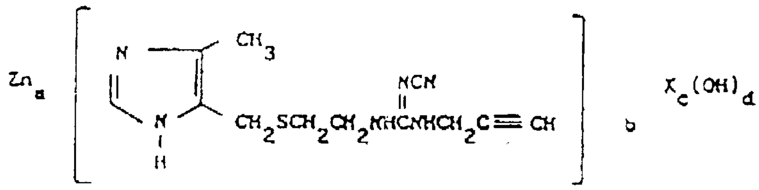 (I)

dans laquelle:
X est un anion d'acides pharmaceutiquement acceptables;
a est un nombre entier de 1 à 5;
b est un nombre entier de 1 à 7;
c vaut 0 ou un nombre entier de 1 à 4; et
d vaut 2a - c pour les anions d'acides monovalents,
a - c pour les anions d'acides bivalents, et
0 ou 2 lorsque c = 0.

2. Dérivés de la revendication 1, caractérisés en ce que X est choisi dans le groupe formé par chlorure, bromure, iodure, fluorure, sulfate, phosphate, chlorate, nitrate, sulfamate, maléate, fumarate, succinate, oxalate, acétate, acéxamate, tartrate, citrate, camphosulfonate, mandélate, butyno-1,4-dioate, benzoate, chlorobenzoate, méthylbenzoate, hydroxybenzoate, méthoxybenzoate, benzènesulfonate, hydroxybenzoate, méthoxybenzoate, benzènesulfonate, toluènesulfonate, phénylacétate, salicylate, bêtahydroxybutyrate, glycolate et méthanesulfonate.

3. Dérivés de la revendication 1, caractérisés en ce que X est l'anion chlorure, a = 1, b = 1, c = 2 et d = 0.

4. Procédé de préparation de dérivés de propargylguanidine de formule générale I:

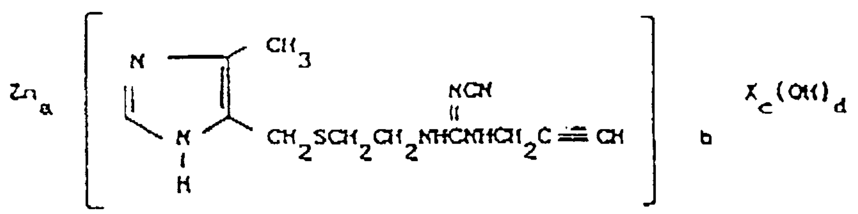 (I)

dans laquelle:
X est un anion d'acides pharmaceutiquement acceptables;
a est un nombre entier de 1 à 5;
b est un nombre entier de 1 à 7;
c vaut 0 ou un nombre entier de 1 à 4; et
d vaut 2a - c pour les anions d'acides monovalents,
a - c pour les anions d'acides bivalents, et
0 ou 2 lorsque c = 0,
caractérisé en ce qu'on fait réagir de la N-cyano-N'-(2-((4-méthyl-5-imidazolyl)méthylthio)éthyl)-N''-propargylguanidine de formule II

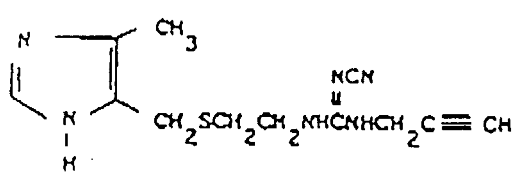 (II)

avec un anion X contenant un composé de zinc ou un organozinc.

5. Procédé de la revendication 4 caractérisé en ce que ledit composé est un anion X contenant un sel de zinc.

6. Procédé de la revendication 4, caractérisé en ce que X est choisi dans le groupe formé par chlorure, bromure, iodure, fluorure, sulfate, phosphate, chlorate, nitrate, sulfamate, maléate, fumarate, succinate, oxalate, acétate, acéxamate, tartrate, citrate, camphosulfonate, mandélate, butyno-1,4-dioate, benzoate, chlorobenzoate, méthylbenzoate, hydroxybenzoate, méthoxybenzoate, benzènesulfonate, toluènesulfonate, phénylacétate, salicylate, bêtahydroxybutyrate, glycolate et méthanesulfonate.

7. Procédé de la revendication 5, caractérisé en ce que ledit sel est le chlorure de zinc.

8. Procédé de l'une quelconque des revendications 1-7, caractérisé en ce que la réaction est conduite dans un solvant polaire.

9. Procédé de la revendication 8, caractérisé en ce que ledit solvant est un alcool de faible poids moléculaire.

10. Procédé de la revendication 9, caractérisé en ce que ledit solvant est choisi dans le groupe formé par le diméthylformamide, le diméthylsulfoxyde, le 1,4-dioxanne, l'acétone ou leurs mélanges aqueux.